(19) **Europäisches Patentamt / European Patent Office / Office européen des brevets**

(11) **EP 4 306 921 B1**

(12) **EUROPEAN PATENT SPECIFICATION**

(45) Date of publication and mention
of the grant of the patent:
**17.12.2025 Bulletin 2025/51**

(51) International Patent Classification (IPC):
**G01J 3/46** *(2006.01)*  **G01N 21/89** *(2006.01)*
**G01N 33/36** *(2006.01)*  *G01N 27/24* *(2006.01)*
**G01N 27/61** *(2006.01)*

(21) Application number: **22184638.9**

(22) Date of filing: **13.07.2022**

(52) Cooperative Patent Classification (CPC):
**G01N 33/365; G01N 21/8915;** G01J 3/463;
G01J 3/501

(54) **ASSESSING THE MANUFACTURING OF A TEXTILE BODY USING COLOR PARAMETERS**

BEWERTUNG DER HERSTELLUNG EINES TEXTILKÖRPERS UNTER VERWENDUNG VON FARBPARAMETERN

ÉVALUATION DE LA FABRICATION D'UN CORPS TEXTILE À L'AIDE DE PARAMÈTRES DE COULEUR

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(43) Date of publication of application:
**17.01.2024 Bulletin 2024/03**

(73) Proprietor: **Gebrüder Loepfe AG**
**8623 Wetzikon (CH)**

(72) Inventors:
• **Hilzinger, Roger**
**8546 Islikon (CH)**
• **Khiar, Amir**
**8405 Winterthur (CH)**
• **Frick, Manuel Bruno**
**8610 Uster (CH)**
• **Schlatter, Adrian**
**8046 Zürich (CH)**

(74) Representative: **E. Blum & Co. AG**
**Franklinturm**
**Hofwiesenstrasse 349**
**8050 Zürich (CH)**

(56) References cited:
**EP-A2- 3 321 668**  **WO-A1-2013/185248**
**WO-A1-99/12019**

• ZHONG ZHILI ET AL: "Comprehensive quality evaluation of jutecell/cotton blended yarn based on principal component analysis", INDIAN JOURNAL OF FIBRE & TEXTILE RESEARCH, vol. 39, 1 September 2014 (2014-09-01), pages 326 - 328, XP093016890
• DORAN ENVER CAN ET AL: "The prediction of quality characteristics of cotton/elastane core yarn using artificial neural networks and support vector machines", TEXTILE RESEARCH JOURNAL, vol. 90, no. 13-14, 27 December 2019 (2019-12-27), pages 1558 - 1580, XP093016886

## Description

<u>Technical Field</u>

The invention relates to a method for assessing the quality of an elongate textile body, in particular of a yarn, as well as to an apparatus to implement such a method.

<u>Background Art</u>

Typically, color components and other body parameters of a yarn or other type of elongate textile bodies, such as yarn-precursors, are measured by means of a sensor head. In order to assess yarn quality, the parameters are monitored to lie inside predetermined ranges. If events are detected where this condition is not met, the sample is determined to be defective.

Defective sections of yarn may e.g. be removed during the rewinding process in a yarn clearer. While such yarn clearing improves overall yarn quality, it leads to loss of time and material.

EP3321668A2 describes a device for measuring optical properties of an elongate body.

Zhong Zhili et al., "Comprehensive quality evaluation of jute-cell/cotton blended yarn based on principal component analysis", Indian Journal of Fibre & Textile Research, Vol. 39, 1 September 2014, pp. 326 - 328 describes a methodology to compare different types of yarns to each other.

<u>Disclosure of the Invention</u>

Hence, it is a general object of the invention to provide a method and apparatus of the type above that allow a better assessment of the quality of the textile body.

This object is achieved by the method and apparatus of the independent claims.

Hence, the invention relates to a method for assessing the quality of an elongate textile body, in particular a yarn, that comprises at least the following steps:

1) Running the elongate textile body past at least one sensor head: The textile body may again e.g. be a yarn precursor, a yarn, or a fabric manufactured from yarn.

2) Determining, by means of the sensor head, a first set of samples $\mathbf{B}_m = (B_{1m} \dots B_{Km})$ of body parameters of the textile body: $K > 1$ is the number of different body parameters in the set, such as the number of different color components. The samples of the body parameters are determined repetitively by means of online measurements, i.e. measurements at one or more locations along the manufacturing chain. m is an index indicative of a time and/or body location a sample $\mathbf{B}_m$ is associated with, with $m = 1 \dots M$ with $M > 1$, advantageously $M > 500$, in particular $M > 100'000$. The samples may be equal to the actually measured values, but typically they are functions thereof. For example, they may be corrected for range, linearity, offset and/or transformed for the values to be normally distributed.

3) Determining "principal components" $\mathbf{w}_n$ of the first set of samples $\mathbf{B}_m$: As known to the skilled person, the "principal components" of a multi-dimensional data set are directions that constitute an orthonormal basis in which different individual dimensions of the data are linearly uncorrelated.

4) Measuring a second set of samples $B'_p = (B'_{1p} \dots B'_{K'p})$ of body parameters for a section of the textile body: This second set of samples may e.g. be a subset of the first set of samples or a different set of samples. The index p ranges from 1 to P, with $P > 0$, i.e. the second set comprises at least one vector of parameters $(B'_{11} \dots B'_{K'1})$, possibly more. Mostly the parameters $1 \dots K'$ are identical to $1 \dots K$ of the first set. However, they may be measured by an identical but distinct sensor. Additionally, they may be distinct functions of the measured values, e.g. the offset subtraction and/or the sampling rate in time or length may differ.

5) For this second set, "deviation scores" $s_{np}$ are determined for each principal component $w_n$ and each sample p. Each deviation score $s_{np}$ is a function D depending at least on the deviation $d_{np}$ along the respective principal component $w_n$ between the sample $B'_p$ and a reference value, advantageously the average of the first or of the second set. The deviation scores $s_{np}$ may e.g. be weighted deviations that are monotonously increasing functions of the deviations $d_{np}$. Alternatively, the deviation scores $s_{np}$ may e.g. be descriptive of a probability of the given deviations $d_{np}$, e.g. based on the respective probability density functions above.

6) Assessing the quality of the section of the textile body using the deviation scores $s_{np}$: By assessing the quality of the section of textile body from the deviation scores $s_{np}$, the quality assessment becomes more robust. Often, textile bodies have a large statistical dispersion along one direction of parameter space, e.g. the color space, and smaller dispersion along another directions of parameter space. However, unusual color variations will catch the eye more than the typical ones. Also, yarn manufacturing is often optimized to cope with variations that occur typically. Hence, variations in typical color directions are of less importance than variations in parameter space that happen less frequently and are therefore "unexpected".

For example, the deviation scores may, for each component, be summed. Alternatively or in addition to this, different predefined weights may be used for differently weighing the deviations along the different principal components, e.g. based on the understanding that some types of defects are less important than other ones.

Advantageously, the method further includes the step of determining dispersion parameters $v_n$ descriptive of the statistical dispersions of the set of samples $\mathbf{B}_m$ along principal components $\mathbf{w}_n$. The "dispersion parameters" describe the extent to which the first set of body parameters or any function thereof are statistically spread along $\mathbf{w}_n$. A dispersion parameter may e.g. be the variance, standard deviation, or a percentile range (e.g. the difference between the 10% and 90% percentiles) of the first set of body along the respective principal component $\mathbf{w}_n$. The dispersion parameters $v_n$ may e.g. also be parameters of the probability density functions PDF along the given principal components.

In that case, function D also depends on the dispersion parameter $v_n$ for the principal components $\mathbf{w}_n$. In this case, function $D(d_{np}, v_n)$ is such that $\partial D/\partial d_{np}$ has opposite sign from $\partial D/\partial vn$. In other words, it weighs the various deviations $d_n$ differently depending on the respective dispersion parameter $v_n$. If the dispersion parameter (i.e. the scatting of the values) is small, a deviation has more weight. For example, $D(d_{pn}, v_n)$ may e.g. be proportional to $d_{np}/v_n$.

Alternatively, i.e. if function D does not depend on the dispersion parameter $v_n$, some fixed weights typical for the processed yarn material may e.g. be used instead.

For example, the color of cotton yarn often has strong variations in normalized luminance but much weaker variations in hue. Hence, a color deviation in hue is more visible than a deviation of the same amplitude in luminance. By weighing deviations in hue more strongly than deviations in luminance, the product quality can therefore be improved.

The method may further comprise the step of explicitly determining at least some of the principal components $\mathbf{w}_n$ from the first set of samples $\mathbf{B}_m$. This may provide further information about the nature of the textile body, e.g. of its composition. It must be noted, though, that there is no need to determine any of the principal components $\mathbf{w}_n$ explicitly in order to determine the dispersion parameters $v_n$. For example, calculating the Mahalanobis norm (see below) allows deriving the dispersion parameters $v_n$ without an explicit knowledge of the principal components $\mathbf{w}_n$.

Advantageously, at least some of the body parameters are color components of the textile body. This allows handling color defects of the textile body.

Alternatively or in addition thereto, the body parameters comprise at least one of the following non-optical parameters:

- a parameter derived from a diameter of the textile body,
- a parameter derived from a capacitance of the textile body, and
- a parameter derived from a triboelectric property of the textile body.

Advantageously, two or more of these non-optical parameters are combined. For example, the linear density, i.e. ratio of capacitance and diameter may be calculated. The linear density may then optionally also be correlated with the triboelectric properties.

This allows correlating non-optical parameters.

Advantageously, the body parameters comprise color components of the textile body as well as at least one of the non-optical parameters. Often, these different types of parameters are strongly correlated. For example, foreign fibers may not only affect the color of the textile body but its diameter or capacitance as well. Hence, the combination of both measures is a better indication for a fault than any of the single measurements taken separately.

In yet another advantageous embodiment, the method may comprise the step of pre-processing the samples using non-linear functions in order to make the statistical distribution of the first set of samples $B_k$ symmetric along the principal components $w_n$ and/or to make the first set of samples $B_k$ have normal distributions. In this way, as described below, "lopsided" dispersions can be corrected.

The methods above may further comprise the step of using the assessed quality for removing a section of textile body in a yarn clearer.

Alternatively or in addition thereto, the methods may further comprise the step of using the assessed quality for automatically displaying an alert to a user.

The present invention also relates to a method for manufacturing a yarn that comprises the method for assessing the quality of an elongate textile body as described herein. The elongate textile body may e.g. be the yarn itself or a yarn precursor.

The method for manufacturing the yarn may also comprise the step of removing a section of yarn as a function of the assessed quality of said section. In other words, the assessed quality may be used in a yarn clearer to test if a given section of yarn has to be removed.

As mentioned, the invention also relates to a yarn manufacturing apparatus. This apparatus comprises a plurality of sensor heads and a control unit adapted and structured to carry out the methods of the present invention.

Brief Description of the Drawings

The invention will be better understood and objects other than those set forth above will become apparent when consideration is given to the following detailed description thereof. This description refers to the annexed drawings, wherein:

Fig. 1 is a schematic view of an apparatus for manufacturing yarn,
Fig. 2 shows an example of the normalized sensitivities of the color components measured by a sensor head,
Fig. 3 is schematic diagram of an embodiment of a sensor head,
Fig. 4 shows the distribution of a measured value (A) and the body parameter (B) derived therefrom, and
Fig. 5 shows an example of the values of a color parameter as measured for textile body.

Modes for Carrying Out the Invention

*Definitions*

*A body parameter* comprises at least two parameters derived from measurements on the elongate body. It may for example include components from a color parameter (see below) and/or non-optical parameters as mentioned herein.

*A color parameter* comprises at least one, in particular several color components of a color space. Advantageously, it includes at least three color components of a color space. The color space is used to describe the color detected by the sensor head. In this context, the color space advantageously spans at least the visible spectrum, e.g. between wavelengths of 380 and 750 nm. It may, however, also extend into the ultraviolet, e.g. at least as low as 250 nm, and/or into the near infrared, e.g. at least as high as 1.8 $\mu$m. Advantageously, the color parameter is descriptive of at least the hue of the color of the textile body, e.g. in HSV color space.

For example, the components may describe the optical reflection or transmission of the textile body in at least two different spectral ranges. Advantageously, there may be at least three color components indicative of the optical reflection or transmission of the textile body in at least three different spectral ranges of the spectrum between, in particular with each of the at least three different colors falling at least in part into a spectral range of 250 nm and 1.8 $\mu$m, in particular at least in part into the visible spectral range of 380 and 750 nm.

The color space may also be the RGB color space or another color space indicative of the color in three or more different spectral ranges, or it may be the CMY or HSV color space, with the components being at least one or two of the coordinates in these color spaces, e.g. the H coordinate.

*A textile body* may be a yarn precursor, in particular a stream of flocks in the blow room, or a sliver or roving as obtained by carding and drawing, or it may be a yarn as obtained by the spinning process or a fabric manufactured from such yarn. In some embodiments, it may also be a product manufactured from the yarn. Advantageously, though, at least the textile body sampled at the claimed sensor head is a yarn.

The *yarn manufacturing apparatus* may at least comprise one of a blow room, a carding machine, a drawing machine, a spinning machine, a winder, and a yarn clearer. Advantageously, the apparatus comprises at least a yarn clearer.

*Overview*

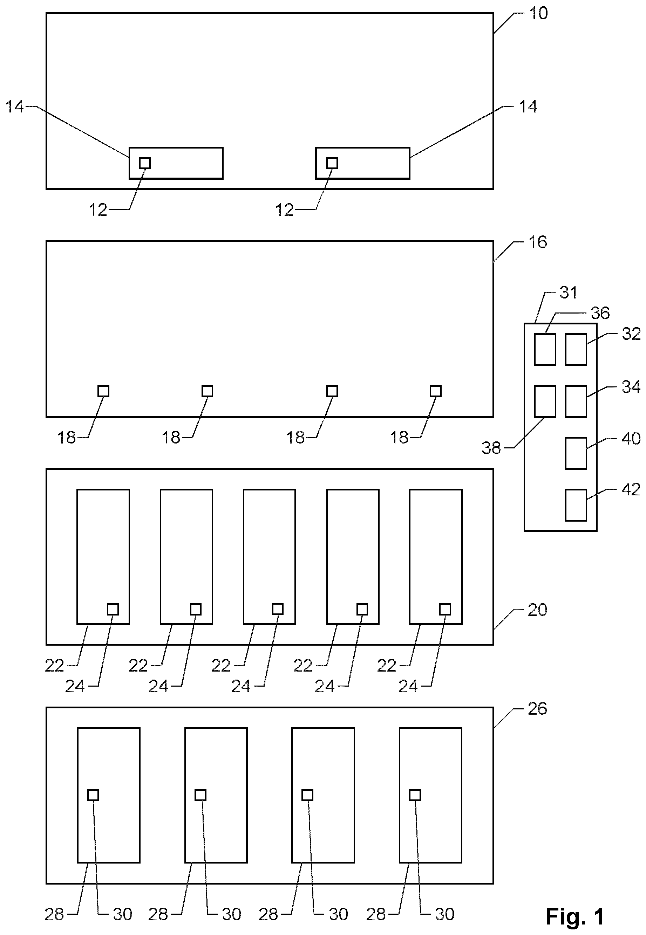

Fig. 1 schematically shows some elements of a cotton spinning mill as an example of a yarn manufacturing apparatus.

As known to the skilled person, such a spinning mill comprises a blow room 10, where bales of cotton are opened into flocks, which may then be precleaned as well as fine cleaned. In this process, one or more streams of flocks are generated.

The stream(s) of flocks may be run past sensor heads 12, e.g. in the fine cleaners 14 of the blow room 10. The function of these sensor heads 12 will be described below.

The mill further comprises a carding and drawing section 16, where the fibers in the flocks are separated, straightened, and formed into slivers and rovings. The slivers or rovings may again be run past sensor heads 18.

In a next step, the products from carding and rowing section 16 are fed to a spinning section 20, where they are spun into yarns in a plurality of spinning units 22. The yarns may again be run past sensor heads 24.

Finally, the yarns, e.g. wound on cops, are fed to a winding section 26 having a plurality of yarn clearers 28. In each yarn clearer 28, the yarn is run past a sensor head 30, e.g. while being wound from cops onto a bobbin.

The apparatus further comprises a control unit 31 controlling the operation of the individual components. In Fig. 1, it is depicted as a single element, but it may also be distributed and/or have sub-sections attributed to the different parts of the apparatus.

Typically, control unit 31 comprises at least one CPU 32, memory 34, input interfaces 36 for receiving sensor signals, e.g. from the various sensor heads as well as from other sensors of the apparatus, output interfaces 38 for controlling actuators of the apparatus, at least one input control 40 for receiving user input, and at least one display 42 for displaying information to the operator.

Control unit 31 may perform various functions using program code and parameters stored in memory 34. In particular, it is programmed to carry out the methods as described herein.

*Sensor heads*

As mentioned above, the apparatus comprises at least one or a plurality of sensor heads 12, 18, 24, 30. Each of these sensor heads is adapted to measure samples $B_{km}$ of one or more of the body parameters of the textile body, where index k = 1 ... K indicates the parameter and index m is indicative of the time and/or yarn position to which the samples are attributed.

The number K of body parameters is advantageously larger than 1. A single sensor head may be adapted to measure all K body parameters, or different sensor heads may be provided to measure different subsets of the body parameters.

The samples of the body parameters can be written as a vector $\mathbf{B}_m = (B_{1m} ... B_{Km})$. They are determined repetitively and online, i.e. they are determined on the textile body while it is being processed by the apparatus in a manufacturing process.

Advantageously, the body parameters include the color components $C_1 ... C_N$ of a color parameter $C = (C_1 ... C_N)$ of the textile body, with N being at least 2, in particular at least 3. Each such color component $C_n$ may describe (i.e. may depend on) the interaction of the textile body with light of a given spectral range $W_n$, wherein the spectral ranges of the different color components differ from each other.

In one embodiment, the color space may be based on different spectral components in a spectral range as specified above.

Advantageously, each color component $C_n$ describes the optical reflectivity of the textile body in the given spectral range. However, in another embodiment, each color component $C_n$ may describe the optical transmission of the textile body at the given spectral range.

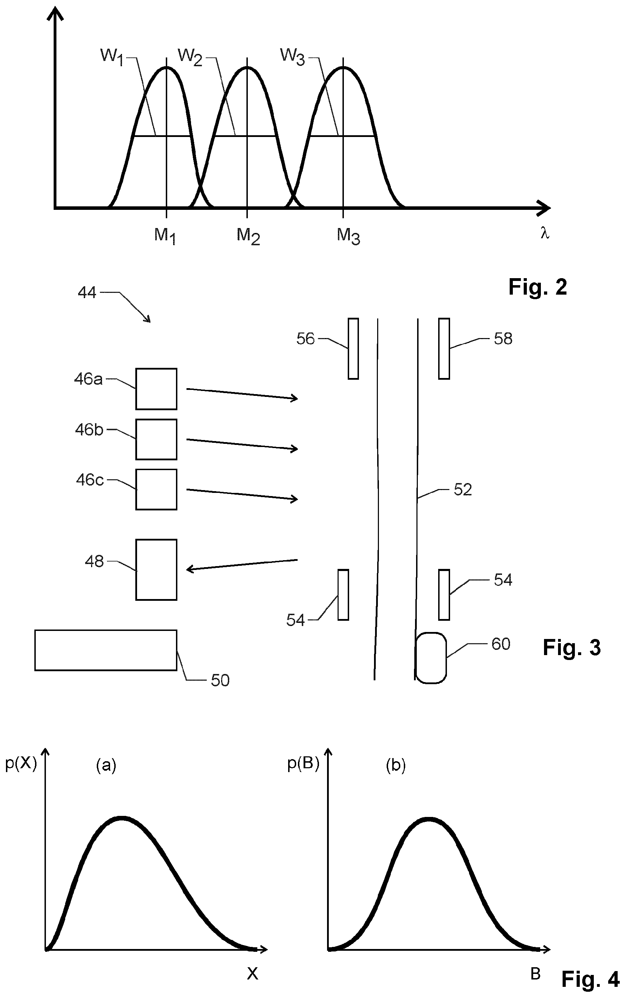

The spectral ranges are advantageously non-overlapping and/or span a substantial part of the visible spectrum. This is illustrated in Fig. 2, which shows the normalized sensitivities of the color components $C_n$ with n = 1 ... N (in this case for N = 3) as a function of the wavelength $\lambda$. For each color component, $W_n$ denotes the spectral range or width at half maximum and $M_n$ the wavelength of maximum sensitivity.

In order to be non-overlapping, there should advantageously be at least two, in particular at least three, color components whose spectral ranges $W_n$ have a mutual overlap by less than 0.25, with the mutual overlap $O_{ij}$ of two spectral ranges $W_i$ and $W_j$ being defined by

$$O_{ij} = 2 * W_{ij} / (W_i + W_j),$$

with $W_{ij}$ being the range where $W_i$ and $W_j$ overlap.

Advantageously, at least one spectral range, in particular at least two of them, should have a width $W_n$ smaller than 50 nm for good color selectivity.

In order to span a substantial part of the visible spectrum, there should advantageously be a least two, in particular at least three, color components whose maximum sensitivities $M_n$ are at least 50 nm away from each other.

Advantageously, at least one maximum sensitivity $M_n$ is in the violet, blue, or green spectral range, i.e. below 550 nm, and at least one maximum sensitivity $M_n$ is in the red spectral range, i.e. above 600 nm. In particular, there is advantageously one maximum sensitivity in the blue or violet spectral range below 500 nm, at least one maximum sensitivity in the green or yellow/orange spectral range between 500 nm and 600 nm, and at least one maximum sensitivity in the red spectral range above 600 nm.

Fig. 3 shows a schematic diagram of a possible embodiment of one sensor head 44, which, in the present embodiment, is adapted to measure color components as well as non-optical parameters of the textile body.

In the shown embodiment, it comprises three light sources 46a, 46b, 46c and a light detector 48. The light sources 46a, 46b, 46c emit light at different spectral ranges, corresponding e.g. to those in Fig. 2. Light sensor 48 is sensitive at all these spectral ranges. Control circuitry 50 is provided for operating the light sources 46a, 46b, 46c to e.g. emit light pulses sequentially and for detecting the response at light sensor 48 for each such light pulse.

The light from the light sources 46a, 46b, 46c falls on the textile body 52 to be inspected, interacts with the same, and, after this interaction, it is detected by light sensor 48. The interaction is advantageously a reflection, i.e. light sensor 48 detects light from the light sources 46a, 46b, 46c that has been reflected from textile body 52.

By attributing the measured light pulses to the individual light sources 46a, 46b, 46c, control circuitry 50 is able to measure the color components $C_n$ for the respective spectral ranges $W_n$.

In yet another embodiment (not shown), three separate light detectors sensitive only in the different spectral ranges $W_n$ may be used, e.g. in combination with a broadband light source.

An example of a suitable sensor head for color components is e.g. described in EP3748343A1.

The sensor head of Fig. 3 may further comprise a capacitive sensor 54, which is adapted to measure the electrical capacitance of a measurement volume comprising textile body 52. Such a sensor is e.g. described in EP3751282A1. This sensor is used to determine a capacitance of the textile body.

The sensor head of Fig. 3 may further comprise a thickness sensor using a light source 56 and a light detector 58 to estimate the diameter of the textile body using shadowing techniques as e.g. described in EP3748342A1.

The sensor head of Fig. 3 may further comprise a triboelectric sensor 60 for measuring a triboelectric property of the textile body, such as e.g. described in CH532526 or US6650959.

*Operation*

In operation, textile body 52 is run past sensor head 44, and sensor head 44 generates measured values $X_{km}$ at times or body locations indexed by index m. The measured values $X_{km}$ may have been subject to preprocessing, e.g. using filtering, downsampling, inverting, offsetting and/or averaging techniques, etc.

A set of samples $\mathbf{B}_m = (B_{1m} \dots B_{Km})$ is determined from the measured values, e.g. using

$$B_{km} = F_k(X_{km}). \tag{1}$$

The functions $F_k$ may be the identity functions, i.e. $B_{km} = X_{km}$, but advantageously, the functions $F_k$ may be selected such that they fulfill at least one of the following conditions:

A) They offset and/or scale the measured values $X_{km}$ to map them into equal numeric ranges for a numerically more stable processing in the following steps.

B) They offset the measured values $X_{km}$ to map them into ranges symmetrical to zero, again to make the following steps easier to implement.

C) The functions $F_k$ may be non-linear, i.e. the samples $B_{km}$ of the body parameters are non-linear transforms of the measurements $X_{km}$. **In** particular, this allows making the statistical distribution of the first set body parameters $B_k$ symmetric, and advantageously even Gaussian, in respect to a center point, e.g. zero. This is illustrated in Fig. 4, where graph (a) depicts an asymmetric probability distribution of a given measurement X and graph (b) depicts the nonlinear transform of X into the body parameter B that has symmetric probability distribution.

Condition C) may also make the distribution along the principal components (see below) symmetric.

Condition C) may e.g. be implemented by:

- Step 1) Writing

$$F_k(X_{km}) = X_{km}{}^a, \tag{2}$$

wherein a is an exponential to be determined by the following steps.

- Step 2a) Calculating, for a plurality of the measurement values $X_{km}$, the values of $F_k(X_{km})$, and then calculating a statistical measure of the asymmetry of the distribution of $F_k(X_{km})$, in particular the skewness, and finding the value of the exponent a where the asymmetry is smallest, or

- Step 2b) Calculating, for a plurality of the measurement values $X_{km}$, the values of $F_k(X_{km})$, performing a statistical normality test on the $F_k(X_{km})$, and finding the value of the exponent a where the distribution of the $F_k(X_{km})$ is closest to a normal distribution.

Steps 2a) and/or 2b) may also be applied to other types of functions $F_k(X_{km})$, in addition to or alternatively to Eq. (2), e.g.

$$F_k(X_{km}) = \log(X_{km}). \tag{2'}$$

In another embodiment, for a set $X_{km}$ with m = 1 to M >> 1, several different quantiles may be calculated and the scale (X-axis) may be mapped nonlinearly to turn the histogram of the set into a normal distribution.

In more general terms, therefore, the present method may comprise the steps of

- measuring, by means of the sensor head, a plurality of measured values $X_{km}$ and
- calculating, from the measured values $X_{km}$, the samples $\mathbf{B}_m$ by means of non-linear transforms $F_k$, wherein the non-linear transforms $F_k$ are adapted to make the distribution of the samples $\mathbf{B}_m$ symmetric, in particular to make said distribution normal.

It must be noted that Eq. (1) assumes that each measured value $X_{km}$ is mapped into a corresponding sample $B_{km}$ of the body parameter. However, preprocessing the measured values may e.g. also comprise the step of combining two or more different measured values $X_{k'm}$, $X_{k"m}$ into a single sample $B_{km}$, i.e.

$$B_{km} = F_k(X_{k'm}, X_{k"m}). \qquad (1')$$

The set of samples determined in this manner can then be used for a plurality of applications described in the following sections.

*Principal component analysis*

In the present method, principal component analysis is used to assess the yarn quality.

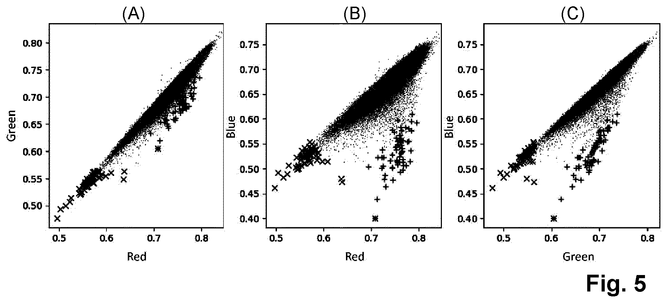

The concept behind this idea is illustrated with reference to Fig. 5, which shows the components of the color parameter measured for a given textile body during a run. In this case, three color components were measured, one for a blue spectral range, one for a green spectral range, and one for a red spectral range. Each one of the dots, +-crosses, and x-crosses in Fig. 5 stands for the color components measured at one time.

As can be seen, the vast majority of the values (the ones that are shown as dots) are confined to a "main" group denoted by dots, but there are also two distinct "outlier" groups, one of which is denoted by the +-crosses and the other one by the x-crosses.

In the present case, the main group corresponds to color values close to the expected, desired color of the body while the outlier groups indicate specific, distinct defects in the body.

As can be seen, though, the main group does not have symmetric distribution. Rather, it is elongate, stretching along the white-black direction (luminance) of the color space. This is a typical behavior e.g. for cotton, where the material may vary in luminance but less in hue and saturation. For this reason, variations in hue or saturation are usually less acceptable than variations in luminance.

Similar issues may arise for other types of materials, such as wool, silk, or synthetic fibers. Especially mélange yarns, as they are a blend of fibers which can have different colors, often show a variation especially between the two colors of their composing fibers.

Therefore, color variations along the elongate axis of the main group may be tolerated while variations perpendicularly thereto should be cleared or flagged more stringently.

While Fig. 5 only shows color components, a similar correlation between other body parameters may also arise.

For example, variations in thickness and capacitance of the textile body are strongly correlated in that a thicker yarn has a larger capacitance. Deviations from this (e.g. thickness varying without the "usual" variation of capacitance) may, however, be indicative of an unusual situation, e.g. due to a variation of the compactness of the yarn or a foreign fiber, that needs flagging or clearing.

Similarly, color components may also be correlated with non-optical body parameters. For example, a dark polyester-white cotton mélange yarn may have strong fluctuations in its luminance combined with a correlated variation of the capacitance between the capacitances of polyester and cotton. A white polyester fault however would combine the typical color of cotton and the capacitance of polyester. Each taken alone, the color and the capacitance, wouldn't be abnormal for the yarn, however, in this combination, they are indicative of a fault.

Hence, advantageously, and as already mentioned above, the present method includes the determination of dispersion parameters $v_n$ that are descriptive of the statistical dispersions of the set of samples $\mathbf{B}_m$ along the principal components $\mathbf{w}_n$ of the set.

In the example of Fig. 5, a first principal component extends substantially along the luminance direction (i.e. between RGB = (0,0,0) and RGB = (1,1,1), while the other two principal components extend perpendicularly thereto.

The dispersion of the body parameters $v_1$ along the first principal component is much larger than the dispersion along the second and third principal component. For the reasons above, variations along the first principal component may be tolerated while variations perpendicularly thereto should be cleared or flagged more stringently.

The dispersion parameters $v_n$ and (if required) the principal components $\mathbf{w}_n$ are advantageously determined from a large set of samples, the "first set of samples" $\mathbf{B}_m = (B_{1m} \dots B_{Km})$. Advantageously, this first set of samples is determined online while manufacturing a certain type of yarn, but it may also be determined offline, i.e. in a separate calibration procedure, and then be reused whenever the same type of yarn is being processed.

The number M of samples $\mathbf{B}_m$ in the first set, with m = 1 ... M, is typically large, in particular M > 500, more advantageously even M > 10000, in order to have a large data set that is robust under statistical analysis.

The dispersion parameters $v_n$ may e.g. correspond to the standard deviation of the first set of samples along principal component $\mathbf{w}_n$, i.e.

$$v_n = \left( \frac{\sum_m ((\mathbf{B}_m - \mathbf{A}) \cdot \mathbf{w_n})^2}{M} \right)^{1/2}, \tag{6}$$

where A is the average value of the first set of samples, and it is assumed that the principal components $\mathbf{w}_n$ are unit vectors.

(Alternatively to the standard deviation, other measures descriptive of the statistical dispersion along the principal components may be used, such as the average deviation or percentiles corresponding to rare events, e.g. 0.02% and 99.8%.)

When the quality of a given yarn section is to be assessed, a "second set of samples" $\mathbf{B}'_p = (B'_{1p} ... B'_{Kp})$ is measured, with p = 1 ... P and P > 0. If the first set of samples is determined online, i.e. during manufacture of the yarn,, the second set of samples may be a subset of or have an intersection with the first set of samples. Otherwise, it may be a distinct set.

For this second set of samples, the raw deviations $d_{np}$ between the samples $\mathbf{B}'_p = (B'_{1p} ... B'_{Kp})$ of the second set and their average are calculated along the principal components $\mathbf{w}_n$.

For example, the deviations $d_{np}$ may be calculated as deviation from the average value A' of the samples, i.e.

$$d_{np} = (\mathbf{B}'_p - \mathbf{A}') \cdot \mathbf{w_n} \tag{7}$$

where $\mathbf{A'}$ is the average value of the second set of samples.

(Alternatively other measures descriptive of the deviation of single points may again be used, e.g. the deviation to the average A instead of A'.)From the raw deviations $d_{np}$, "deviation scores" $s_{np}$ can be calculated using

$$s_{np} = D(d_{np}, v_n). \tag{8}$$

Advantageously, as mentioned, function D is such that $\partial D/\partial d_{np}$ has opposite sign from $\partial D/\partial v_n$. In other words, it weighs the various deviation $d_{np}$ differently depending on the respective dispersion parameter $v_n$. For example,

$$D(d_{np}, v_n) = k \cdot (d_{np} / v_n)^i, \tag{9}$$

with i being an exponent > 0, e.g. i = 1, and with k being a constant, that may be settable by the user but advantageously is not and advantageously is the same for all n.

Finally, the quality of the section of textile body can then be assessed from these deviation scores $s_{np}$.

For example, similar as in the example of dye color above, a fault score Q may be calculated that is indicative of the likelihood that a given section of textile body is a defect. For example, the fault score Q may be given by the sum of the squared deviation scores $s_{np}$.

Advantageously, Q is the same for all $\mathbf{B}'_p$ sharing the same probability to occur. Therefore, since the deviations have already been weighted, the fault score Q is advantageously equally dependent on all deviation scores $s_n$, i.e.

$$\partial Q/\partial s_{ip} = \partial Q/\partial s_{jp} \tag{10}$$

for all i, j.

Advantageously, the weight of the single $s_{np}$ in the calculation of Q can however be modified by the user. This is e.g. useful if a yarn is a blend of dark man-made fibers and shorter white cotton fibers. The longer fibers are mainly responsible for the strength of the yarn. Therefore, while for the appearance of the fabric all $s_{np}$ are equally important, the user may want to be more severe along the dimension representing the blend ratio, attributing a higher weight to it.

Hence, in that case, the method advantageously comprises the step of providing a user interface for selecting a fault score parameter $p_n$ for each principal component. The dependence of the fault score Q on each of the deviation scores, i.e. $\partial Q/\partial s_{ip}$, depends on the respective fault score parameter $p_i$. At least some of the fault score parameters $p_i$ differ

EP 4 306 921 B1

from each other.

Instead of calculating the fault score Q as described above, the fault score can e.g. also be calculated from the Mahalanobis distance, see e.g. https://en.wikipedia.org/wiki/Mahalanobis_distance, which uses the covariance matrix of the first set of samples and does not rely on an explicit calculation of the principal components even though it is still considered to be a method of principal component analysis.

Principal component analysis is advantageously combined with pre-processing the samples as described in the section "Operation" above using non-linear functions $F_k$ in order to make the statistical distribution of the first set samples $B_k$ symmetric, and advantageously even normal (Gaussian).

Instead of applying such non-linear functions $F_k$ on the measured values $X_{km}$ directly, non-linear transforms may also be applied to the samples $\mathbf{B}_m$ or when calculating the deviations $d_{np}$ or the deviation scores $s_{np}$.

In the above examples, the deviation scores $s_{np}$ are weighted deviations that are monotonously increasing functions of the deviations $d_{np}$. Alternatively, though, they may also be descriptive of the probability of the deviations $d_{np}$, in which case they are typically monotonously decreasing functions of the deviations $d_{np}$.

Such probabilities may e.g. be calculated from the probability density function $PDF_n(d_{np})$, which describes the relative likelihood that the value of the parameter would have a deviation close to the deviations $d_{np}$, see e.g. https://en.wikipedia.org/wiki/Probability_density_function.

*Notes*

In the present system, the quality of an elongate textile body 52, such as a yarn precursor or a yarn, is assessed. The textile body is run past a sensor head 20, 18, 24, 30; 44. At a first plurality of times, samples of the body parameters of the textile body 52 are measured. The parameters may include color components and/or other parameters, such as the thickness, capacitance, or triboelectric properties of the body.

Principal component analysis may be used to scale deviations in parameter space to make the quality assessment more robust in the presence of non-isotropic distribution of the samples.

A minimum variation of the parameters may be requested when assessing the quality of mélange yarns.

The color of a dye to be applied to the manufactured yarn may be taken into account in order to reduce the number of flagged or cleared yarn sections.

While there are shown and described presently preferred embodiments of the invention, it is to be distinctly understood that the invention is not limited thereto but may be otherwise variously embodied and practiced within the scope of the following claims.

**Claims**

1. A method for assessing quality of an elongate textile body comprising the steps of

   - running the elongate textile body (52) past at least one sensor head (12, 18, 24, 30; 44),
   - determining, by means of the sensor head (12, 18, 24, 30; 44), a first set of samples $\mathbf{B}_m = (B_{1m} \ldots B_{Km})$ of body parameters of the textile body (52) with K > 1 by repetitively carrying out online measurements, wherein the first set of samples $\mathbf{B}_m$ is derived from the measurements, and wherein m = 1 ... M with M > 1,
   - measuring a second set of samples $\mathbf{B}'_p = (B'_{1p} \ldots B'_{Kp})$ of body parameters for a section of the textile body, wherein p = 1 ... P with P > 0,

   **characterized by** the steps of

   - determining principal components $\mathbf{w}_n$ of the first set of samples $\mathbf{B}_m$,
   - determining deviation scores $s_{np}$ for each principal component $\mathbf{w}_n$, wherein each deviation score $s_{np}$ is a function D depending at least on a deviation $d_{np}$ between the second and first set along the respective principal component $\mathbf{w}_n$, and
   - assessing the quality of the section of the textile body using the deviation scores $s_{np}$.

2. The method of claim 1 further comprising the step of determining dispersion parameters $v_n$ descriptive of dispersions of the first set of samples $\mathbf{B}_m$ along the principal components $\mathbf{w}_n$, wherein the function D further depends on the dispersion parameter $v_n$ for the principal component wn, wherein $\partial D/\partial d_{np}$ has opposite sign from $\partial D/\partial v_n$.

3. The method of claim 2 wherein the dispersion parameters $v_n$ are parameters of probability distribution functions along

9

the principal components and wherein the deviation scores are descriptive of a probability of the given deviations $d_{np}$ according to the respective probability distribution functions.

4. The method of any of the preceding claims comprising the step of determining at least some of the principal components $\mathbf{w}_n$ from the first set of samples $\mathbf{B}_m$.

5. The method of any of the preceding claims wherein the second set of samples $\mathbf{B'}_p$ is a subset of the first set of samples $\mathbf{B}_m$.

6. The method of any of the preceding claims where at least some of said body parameters are color components of the textile body (52).

7. The method of any of the preceding claims wherein the body parameters comprise at least one of

a parameter derived from a diameter of the textile body,
a parameter derived from a capacitance of the textile body, and
a parameter derived from a triboelectric property of the textile body,

8. The method of any of the preceding claims comprising the step of pre-processing the samples using non-linear functions ($F_k$), wherein the non-linear functions ($F_k$) are adapted to make a distribution of the first set of samples along the principal components $\mathbf{w}_n$ symmetric and/or to make the first set of samples $B_k$ have normal distributions.

9. The method of any of the preceding claims comprising the step of calculating a fault score Q from the deviation scores $s_{np}$,

10. The method of claim 9 comprising the step of
providing a user interface for selecting a fault score parameter for each principal component, wherein the dependence of the fault score Q on each of the deviation scores $s_{np}$ depends on the respective fault score parameter.

11. The method of claim 9 wherein the fault score Q is equally dependent on all deviation scores $s_{np}$.

12. The method of any of the preceding claims comprising the steps of

measuring, by means of the sensor head (12, 18, 24, 30; 44), a plurality of measured values ($X_{km}$) and calculating, from the measured values ($X_{km}$), the samples $\mathbf{B}_m$ by means of non-linear transforms ($F_k$), wherein the non-linear transforms ($F_k$) are adapted to make a distribution of the samples $\mathbf{B}_m$ symmetric, in particular to make said distribution normal.

13. The method of any of the preceding claims comprising the step of using the assessed quality for automatically displaying an alert to a user.

14. A method for manufacturing a yarn comprising the method of any of the preceding claims.

15. The method of claim 14 comprising the steps of

comparing a color parameter (Cp) in said section against a dye color (Cd) indicative of the color of a dye the elongate textile body (52) is to be dyed with, and
dyeing the yarn with the dye having the dye color (Cd).

16. The method of any of the claims 14 or 15 comprising the step of removing a section of yarn as a function of the assessed quality of said section.

17. A yarn manufacturing apparatus comprising

at least one sensor head (12, 18, 24, 30; 44) and
**characterized by** a control unit (31) adapted and structured to carry out the method of any of the preceding claims.

**Patentansprüche**

1.  Verfahren zur Bewertung der Qualität eines länglichen Textilkörpers, umfassend die folgenden Schritte

    - Führen des länglichen Textilkörpers (52) an mindestens einem Sensorkopf (12, 18, 24, 30; 44) vorbei,
    - Bestimmen, mittels des Sensorkopfes (12, 18, 24, 30; 44), eines ersten Satz von Messwerten $\mathbf{B}_m = (B_{1m} ... B_{Km})$ von Körperparametern des Textilkörpers (52), mit K > 1, durch wiederholte Durchführung von Online-Messungen, wobei der erste Satz von Messwerten $\mathbf{B}_m$ aus den Messungen abgeleitet wird und wobei m = 1 ... M mit M > 1 ist,
    - Messen eines zweiten Satzes von Messwerten $\mathbf{B'}_p = (B'_{1p} ... B'_{Kp})$ von Körperparametern des Textilkörpers, wobei p = 1 ... P mit P > 0,
    **gekennzeichnet durch** die Schritte
    - Bestimmen von Hauptkomponenten $\mathbf{w}_n$ des ersten Satzes von Messwerten $\mathbf{B}_m$,
    - Bestimmen von Abweichungs-Scores $s_{np}$ für jede Hauptkomponente $\mathbf{w}_n$, wobei jeder Abweichungs-Score $s_{np}$ eine Funktion D ist, die mindestens von einer Abweichung $d_{np}$ zwischen dem zweiten und dem ersten Satz entlang der jeweiligen Hauptkomponente $\mathbf{w}_n$ abhängt, und
    - Bewerten der Qualität des Abschnitts des Textilkörpers unter Verwendung der Abweichungs-Scores $s_{np}$.

2.  Verfahren nach Anspruch 1, das ferner den Schritt umfasst, Verteilungsparameter $v_n$ zu bestimmen, die die Verteilungen des ersten Satzes von Messwerten $\mathbf{B}_m$ entlang der Hauptkomponenten $\mathbf{w}_n$ beschreiben, wobei die Funktion D ferner vom Verteilungsparameter $v_n$ für die Hauptkomponente wn abhängt, wobei $\partial D/\partial d_{np}$ das entgegengesetzte Vorzeichen von $\partial D/\partial v_{(n)}$ hat.

3.  Verfahren nach Anspruch 2, wobei die Verteilungsparameter $v_n$ Parameter von Wahrscheinlichkeitsverteilungsfunktionen entlang der Hauptkomponenten sind und wobei die Abweichungs-Scores eine Wahrscheinlichkeit der gegebenen Abweichungen $d_{np}$ gemäss den jeweiligen Wahrscheinlichkeitsverteilungsfunktionen beschreiben.

4.  Verfahren nach einem der vorstehenden Ansprüche, das den Schritt des Bestimmens mindestens einiger der Hauptkomponenten $_{Wn}$ aus dem ersten Satz von Messwerten $\mathbf{B}_m$ umfasst.

5.  Verfahren nach einem der vorstehenden Ansprüche, wobei der zweite Satz von Messwerten $\mathbf{B'}_p$ eine Teilmenge des ersten Satzes von Messwerten $\mathbf{B}_m$ ist.

6.  Verfahren nach einem der vorstehenden Ansprüche, wobei zumindest einige der Körperparameter Farbkomponenten des Textilkörpers (52) sind.

7.  Verfahren nach einem der vorstehenden Ansprüche, wobei die Körperparameter mindestens einen der folgenden Parameter umfassen

    einen aus einem Durchmesser des Textilkörpers abgeleiteten Parameter,
    einem aus einer Kapazität des Textilkörpers abgeleiteten Parameter,
    und
    einem aus einer triboelektrischen Eigenschaft des Textilkörpers abgeleiteten Parameter.

8.  Verfahren nach einem der vorstehenden Ansprüche, umfassend den Schritt der Vorverarbeitung der Messwerte unter Verwendung nichtlinearer Funktionen ($F_k$), wobei die nichtlinearen Funktionen ($F_k$) so ausgestaltet sind, dass sie eine Verteilung der ersten Gruppe von Messwerten entlang der Hauptkomponenten $\mathbf{w}_n$ symmetrisch machen und/oder die erste Gruppe von Messwerten $B_k$ normalverteilt machen.

9.  Verfahren nach einem der vorstehenden Ansprüche, umfassend den Schritt des Berechnens einer Fehlerbewertung Q aus den Abweichungs-Scores $s_{np}$.

10. Verfahren nach Anspruch 9, umfassend den Schritt
    Bereitstellen einer Benutzerschnittstelle zum Auswählen eines Fehlerbewertungsparameters für jede Hauptkomponente, wobei die Abhängigkeit der Fehlerbewertung Q von jedem der Abweichungs-Scores $s_{np}$ von dem jeweiligen Fehlerbewertungsparameter abhängt.

11. Verfahren nach Anspruch 9, wobei die Fehlerbewertung Q gleichermassen von allen Abweichungs-Scores $s_{np}$

abhängt.

**12.** Verfahren nach einem der vorstehenden Ansprüche, das die folgenden Schritte umfasst

Messen einer Vielzahl von Messwerten ($X_{km}$) mittels des Sensorkopfes (12, 18, 24, 30; 44) und Berechnen der Messwerten $\mathbf{B}_m$ aus den Messwerten ($X_{km}$) mittels nichtlinearer Transformationen ($F_k$), wobei die nichtlinearen Transformationen ($F_k$) so ausgelegt sind, dass sie eine Verteilung der Abtastwerte $\mathbf{B}_m$ symmetrisch machen, insbesondere dass sie diese Verteilung normalverteilt machen.

**13.** Verfahren nach einem der vorstehenden Ansprüche, umfassend den Schritt des Verwendens der bewerteten Qualität zum automatischen Anzeigen einer Warnung für einen Benutzer.

**14.** Verfahren zur Herstellung eines Garns umfassend das Verfahren nach einem der vorstehenden Ansprüche.

**15.** Verfahren nach Anspruch 14, umfassend die Schritte

Vergleichen eines Farbparameters (Cp) in dem Abschnitt mit einer Farbstofffarbe (Cd), die die Farbe eines Farbstoffs angibt, mit dem der längliche Textilkörper (52) gefärbt werden soll, und Färben des Garns mit dem Farbstoff, der die Farbstofffarbe (Cd) aufweist.

**16.** Verfahren nach einem der Ansprüche 14 oder 15, umfassend den Schritt des Entfernens eines Abschnitts des Garns in Abhängigkeit von der bewerteten Qualität des Abschnitts.

**17.** Garnherstellungsvorrichtung, umfassend

mindestens einen Sensorkopf (12, 18, 24, 30; 44) und **gekennzeichnet durch** eine Steuereinheit (31), die dazu ausgelegt und aufgebaut ist, das Verfahren nach einem der vorstehenden Ansprüche durchzuführen.

## Revendications

**1.** Un procédé pour évaluer la qualité d'un corps textile allongé, comprenant les étapes suivantes

- faire passer le corps textile allongé (52) devant au moins une tête de capteur (12, 18, 24, 30 ; 44),
- déterminer, à l'aide de la tête de capteur (12, 18, 24, 30 ; 44), un premier ensemble de valeurs mesurées $\mathbf{B}_m = (B_{1m}... B_{Km})$ de paramètres du corps du corps textile (52), avec K > 1, en effectuant de manière répétée des mesures en ligne, dans lequel le premier ensemble de valeurs mesuré $\mathbf{B}_m$ est dérivé des mesures et m = 1 ... M avec M > 1,
- mesurer un deuxième ensemble de valeurs mesurées $\mathbf{B'}_p = (B'_{1p}... B'_{Kp})$ de paramètres du corps textile, où p = 1... $\mathbf{P}$ avec $\mathbf{P}$ > 0,

**caractérisé par** les étapes suivantes

- déterminer des composantes principales $\mathbf{w}_n$ du premier ensemble de valeurs mesurées $\mathbf{B}_m$,
- déterminer de scores de déviation $s_{np}$ pour chaque composante principale $\mathbf{w}_n$, dans lequel chaque score d'écart $s_{np}$ est une fonction D, qui dépend au moins d'un écart $d_{np}$ entre le deuxième et le premier ensemble le long de la composante principale respective $\mathbf{w}_n$, et
- évaluer la qualité de la section du corps textile à l'aide des scores d'écart $s_{np}$.

**2.** Le procédé selon la revendication 1, comprenant en outre l'étape consistant à déterminer des paramètres de dispersion $v_n$ qui décrivent les dispersions du premier ensemble de valeurs mesurées $\mathbf{B}_m$ le long des composantes principales $\mathbf{w}_n$, dans lequel la fonction D dépend en outre du paramètre de dispersion $v_n$ pour la composante principale wn, dans lequel $\partial D/\partial d_{np}$ a le signe opposé à $\partial D/\partial v_n$.

**3.** Le procédé selon la revendication 2, dans lequel les paramètres de dispersion $v_n$ sont des paramètres de fonctions de distribution de probabilité le long des composantes principales et dans lequel les scores d'écart décrivent une probabilité des déviations donnés $d_{np}$ selon les fonctions de distribution de probabilité respectives.

4. Le procédé selon l'une des revendications précédentes, qui comprend l'étape consistant à déterminer au moins certaines des composantes principales $w_n$ à partir du premier ensemble de valeurs mesurées $B_m$.

5. Le procédé selon l'une des revendications précédentes, dans lequel le deuxième ensemble de valeurs mesurées $B'_p$ est un sous-ensemble du premier ensemble de valeurs mesurées $B_m$.

6. Le procédé selon l'une des revendications précédentes, dans lequel au moins certains des paramètres de corps sont des composantes de couleur du corps textile (52).

7. Le procédé selon l'une des revendications précédentes, dans lequel les paramètres de corps comprennent au moins l'un des paramètres suivants

   un paramètre dérivé d'un diamètre du corps textile,
   un paramètre dérivé d'une capacité du corps textile,
   et
   un paramètre dérivé d'une propriété triboélectrique du corps textile.

8. Le procédé selon l'une des revendications précédentes, comprenant l'étape de prétraiter les valeurs mesurées à l'aide de fonctions non linéaires ($F_k$), dans lequel les fonctions non linéaires ($F_k$) sont conçues de manière à rendre symétrique une distribution du premier groupe de valeurs mesurées le long des composantes principales $w_n$ et/ou à rendre le premier groupe de valeurs mesurées $B_k$ avoir des distributions normales.

9. Le procédé selon l'une des revendications précédentes, comprenant l'étape consistant à calculer une évaluation d'erreur $Q$ à partir des scores de déviation $s_{np}$.

10. Le procédé selon la revendication 9, comprenant l'étape de
    fournir une interface utilisateur pour sélectionner un paramètre d'évaluation d'erreur pour chaque composante principale, dans lequel la dépendance de l'évaluation d'erreur Q de chacun des scores de déviation $s_{np}$ dépend du paramètre d'évaluation d'erreur respectif.

11. Le procédé selon la revendication 9, dans lequel l'évaluation d'erreur Q dépend de manière égale de tous les scores de déviation $s_{np}$.

12. Le procédé selon l'une des revendications précédentes, comprenant les étapes suivantes

    mesurer une pluralité de valeurs mesurées ($X_{km}$) à l'aide de la tête de capteur (12, 18, 24, 30 ; 44) et
    calculer des valeurs mesurées $B_m$ à partir des valeurs mesurées ($X_{km}$) au moyen de transformations non linéaires ($F_k$), dans lequel les transformations non linéaires ($F_k$) sont conçues de manière à rendre symétrique une distribution des valeurs échantillonnées $B_m$, en particulier à rendre cette distribution normale.

13. Le procédé selon l'une des revendications précédentes, comprenant l'étape consistant à utiliser la qualité évaluée pour afficher automatiquement un avertissement à un utilisateur.

14. Un procédé pour fabriquer un fil comprenant le procédé selon l'une des revendications précédentes.

15. Le procédé selon la revendication 14, comprenant les étapes

    comparer un paramètre de couleur **(Cp)** dans la section avec une couleur de colorant **(Cd)** indiquant la couleur d'un colorant avec lequel le corps textile allongé (52) doit être teint, et
    teindre le fil avec le colorant ayant la couleur de colorant (**Cd**).

16. Le procédé selon l'une des revendications 14 ou 15, comprenant l'étape consistant à retirer une partie du fil en fonction de la qualité évaluée de cette partie.

17. Un dispositif de fabrication de fil comprenant

au moins une tête de capteur (12, 18, 24, 30 ; 44)
et
**caractérisé par** une unité de commande (31) qui est conçue et construite pour mettre en œuvre le procédé selon l'une des revendications précédentes.

EP 4 306 921 B1

Fig. 1

15

**Fig. 2**

**Fig. 3**

**Fig. 4**

(A) (B) (C)

Fig. 5

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 3321668 A2
- EP 3748343 A1
- EP 3751282 A1

- EP 3748342 A1
- CH 532526
- US 6650959 B

**Non-patent literature cited in the description**

- **ZHONG ZHILI et al.** Comprehensive quality evaluation of jute-cell/cotton blended yarn based on principal component analysis. *Indian Journal of Fibre & Textile Research*, 01 September 2014, vol. 39, 326-328